# EUROPEAN PATENT APPLICATION

(11) **EP 4 670 755 A1**
(43) Date of publication of application: **31.12.2025**
(21) Application number: 25185004.6
(22) Date of filing: 24.06.2025
(51) Int. Cl.: A61M 1/36

(54) **BLOOD PURIFICATION DEVICE AND METHOD FOR PRIMING BLOOD CIRCULATION PASSAGE**

(30) Priority: 28.06.2024 JP 2024105575
(71) Applicant: Shibuya Corporation, Kanazawa-shi, Ishikawa 920-8681 (JP)
(72) Inventor: MATSUZAKI, Kosho, Kanazawa-shi, 920-8681 (JP); FUJII, Masayuki, Kanazawa-shi, 920-8681 (JP)
(74) Representative: Mewburn Ellis LLP

(57) **Abstract**

To reduce the workload of medical personnel, and suppress the use of dialysate as priming fluid.

A supply port P1 is provided in a dialysate supply passage 34 and a discharge port P3 is provided in a dialysate discharge passage 35. A supply passage 23 is branched on an upstream side of a blood pump 8 in the arterial passage 21, and a venous passage connection part (branch passage 24) is provided in the supply passage 23 to which a venous passage 22 can be connected.

When priming, with the supply passage 23 connected to the supply port P1, the arterial passage 21 to the discharge port P3, and the venous passage 22 to the branch passage 24 of the supply passage 23, respectively, the dialysate supply device and the blood pump 8 are actuated to simultaneously cause dialysate to flow through the arterial passage 21 and the venous passage 22 to prime the blood circulation passage consisting of a blood purifier 2 and a blood circuit 3.

## Description

### BACKGROUND OF THE INVENTION

### Field of the Invention

The present invention relates to a blood purification device and a method for priming a blood circulation passage, and in particular to a blood purification device and a method for priming a blood circulation passage in which dialysate is supplied from a dialysate circuit to a blood circuit for priming.

### Description of the Related Art

A blood purification device used for blood purification treatment such as hemodialysis includes a blood circuit having an arterial passage and a venous passage connected to a blood purifier and a dialysate circuit having a dialysate supply passage and a dialysate discharge passage connected to the blood purifier, so that blood is purified in the blood purifier while circulating outside the body (Japanese Laid-Open Patent Application No. 2024-35694).

In such a blood purification device, it is necessary to replace the blood purifier and blood circuit with a new blood purifier and blood circuit each time blood purification treatment is performed, and when a new blood purifier and blood circuit are used, priming is required to be performed to remove air inside by circulating priming fluid through the blood circulation passage consisting of the blood purifier and blood circuit.

In conventional blood purification devices, dialysate is supplied from the dialysate circuit to the blood circuit as priming fluid during the above priming, and, in the blood purification device of Japanese Laid-Open Patent Application No. 2024-35694 above, the dialysate is supplied using an infusion passage provided between the dialysate supply passage of the dialysate circuit and the blood circuit.

The blood purification device of Japanese Laid-Open Patent Application No. 2024-35694 is designed to select either pre-infusion (pre-dilution method), in which supplemental fluid is supplied to the arterial passage of the blood circuit or post-infusion (post-dilution method), in which supplemental fluid is supplied to the venous passage, depending on the patient's condition and the physician's judgment, and priming is performed in a blood circuit where the infusion passage is branched from either the arterial passage or the venous passage.

When blood purification treatment is performed with the blood purification device of Japanese Laid-Open Patent Application No. 2024-35694 above, the blood purifier is held so that the arterial side header (red) to which the arterial passage of the blood circuit is connected is above and the venous side header (blue) to which the venous passage is connected is below, and blood is caused to flow from above to below inside the hollow fiber membrane consisting of many hollow fibers installed inside the blood purifier.

In contrast, priming of blood purifiers requires priming fluid to be caused to flow from below to above inside the hollow fiber membrane. In other words, when the priming fluid is caused to flow from above to below inside the hollow fiber membrane, some air may rise against the flow of the priming fluid and remain in the header above, and air must be allowed to flow out of the blood purifier together with the priming fluid by causing the priming fluid to flow from below to above inside the hollow fiber membrane to facilitate air removal.

Here, in Japanese Laid-Open Patent Application No. 2024-35694 above, blood is caused to flow from above to below with the arterial side header up during treatment. This allows the blood purifier to prime by causing the priming fluid to flow from below to above with the venous side header facing downward, because the blood circuit with the infusion passage branched from the venous passage is used when post-infusion is selected and dialysate is supplied to the venous passage for priming, and the arterial side header is upward, allowing the patient to shift directly to treatment.

However, if pre-infusion is selected, since a blood circuit with an infusion passage branched from the arterial passage is used, and since dialysate is supplied to the arterial passage during priming, the blood purifier must be held with the arterial side header facing downward to cause the priming fluid to flow from below to above.

Therefore, in order to perform treatment after priming, the blood purifier had to be inverted by medical personnel so that the arterial side header was up.

Thus, in Japanese Laid-Open Patent Application No. 2024-35694, the transition from priming to treatment sometimes requires work, and there was a risk of a human error because the operations were different between post-infusion and pre-infusion.

In addition, in the blood purification device of Japanese Laid-Open Patent Application No. 2024-35694, when priming the hollow fiber membrane of the blood purifier, all the dialysate that had passed through the hollow fiber membrane was drained without being circulated, and a large amount of dialysate was used to sufficiently remove air.

In view of these problems, the present invention provides a blood purification device and a method for priming a blood circulation passage, which can reduce the workload of medical personnel, prevent the occurrence of a human error, and control the amount of dialysate used in priming.

### SUMMARY OF THE INVENTION

A blood purification device according to the invention of claim 1 is a blood purification device having a blood circuit having an arterial passage and a venous passage connected to a blood purifier, a dialysate circuit having a dialysate supply passage and a dialysate discharge passage connected to the blood purifier, dialysate supply device for supplying dialysate to the blood purifier via the dialysate supply passage, a blood pump installed in the arterial passage to pump blood to the blood purifier, and control device for controlling operation of the dialysate supply device and the blood pump, the blood purification device being characterized by comprising:
a supply port for causing dialysate to flow out of the dialysate supply passage and a discharging port for causing dialysate to flow into the dialysate discharge passage; and
a supply passage branching from an upstream side of the blood pump in the arterial passage and a venous passage connection part that is connectable to the venous passage in the middle of the supply passage,
and characterized in that, when priming the blood circulation passage consisting of the blood purifier and blood circuit,
with the supply passage connected to the supply port, the arterial passage connected to the discharge port, and the venous passage connected to the venous passage connection part of the supply passage,
the control device actuates the dialysate supply device and the blood pump to supply dialysate from the dialysate supply passage to the arterial passage via the supply passage and further circulates the dialysate from the arterial passage through the blood purifier and venous passage, and causes the dialysate to flow out from the arterial passage to the dialysate discharge passage.
A method of priming a blood circulation passage according to the invention of claim 3 is a method of priming a blood circulation passage including a blood purifier and a blood circuit in a blood purification device having the blood circuit having an arterial passage and a venous passage connected to the blood purifier, a dialysate circuit having a dialysate supply passage and a dialysate discharge passage connected to the blood purifier, dialysate supply device for supplying dialysate to the blood purifier via the dialysate supply passage, a blood pump installed in the arterial passage to pump blood to the blood purifier, and control device for controlling operation of the dialysate supply device and the blood pump, the method being characterized by comprising:
connecting an upstream side of the blood pump in the dialysate supply passage and the arterial passage via a supply passage, connecting the venous passage to a middle of the supply passage, and connecting the arterial passage to the dialysate discharge passage, and
supplying the dialysate from the dialysate supply passage to the arterial passage via the supply passage by the dialysate supply device and causing the dialysate to be circulated by the blood pump from the arterial passage through the blood purifier and the venous passage and causing the dialysate to be discharged from the arterial passage to the dialysate discharge passage.

According to the invention of claims 1 and 3 above, during priming, since dialysate is supplied to an upstream side of the blood pump in the arterial passage from the dialysate supply passage via the supply passage as priming fluid, the dialysate is supplied to the blood purifier from below.

During blood purification treatment, blood is supplied to the blood purifier from below, and so there is no need to invert the blood purifier prior to blood purification treatment, and this can reduce workload on medical personnel and prevent the occurrence of a human error.

Also, by connecting the venous passage to middle of the supply passage, a circulation passage through the blood purifier can be formed for performing priming, thereby suppressing the amount of dialysate used.

### BRIEF DESCRIPTION OF THE DRAWINGS

FIG. 1 is a front view of a blood purification device according to an embodiment;
FIG. 2 is a diagram explaining an operation for priming in the blood purification device;
FIG. 3 is a diagram explaining an operation for priming in the blood purification device;
FIG. 4 is a diagram explaining an operation for priming in the blood purification device;
FIG. 5 is a diagram explaining an operation for priming in the blood purification device; and
FIG. 6 is a diagram explaining an operation for priming in a case where the blood purification device is used in pre-infusion.

### DETAILED DESCRIPTION OF THE INVENTION

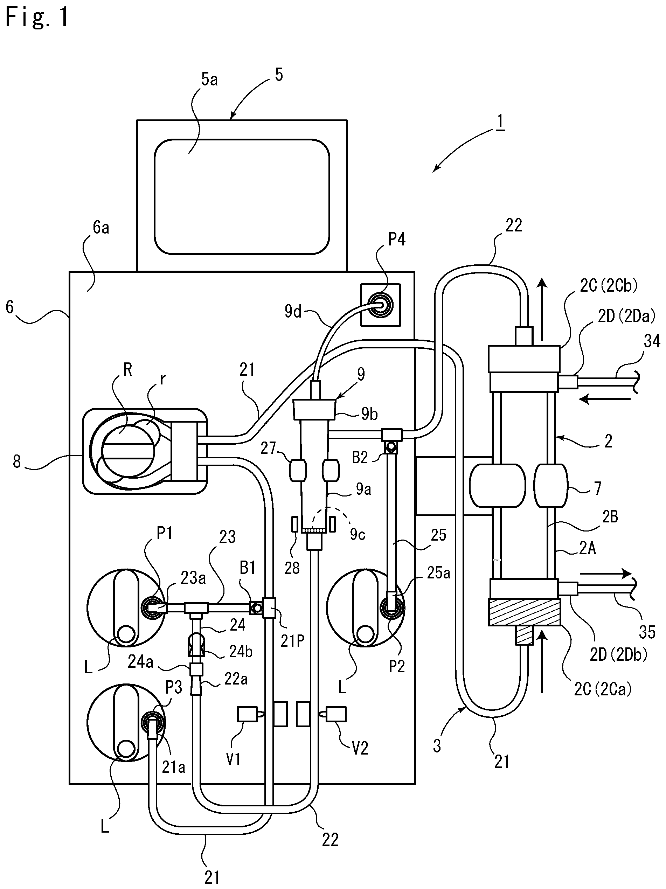

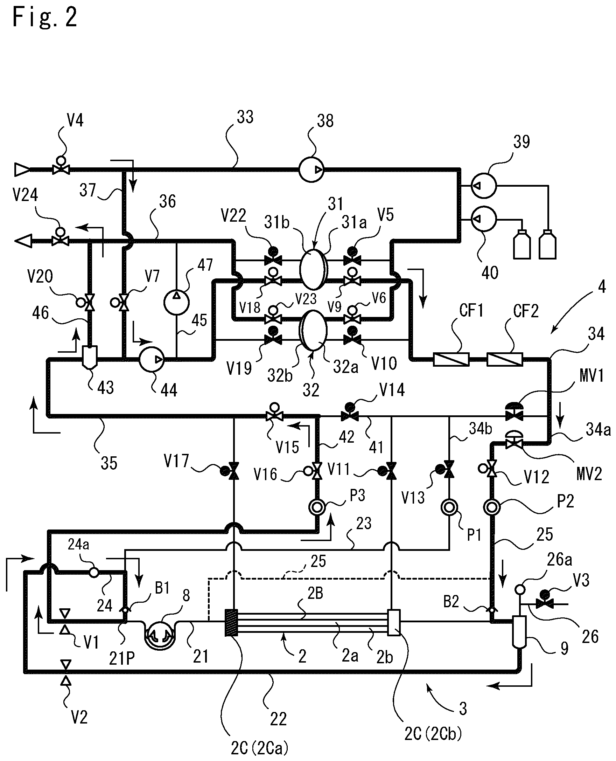

With reference to FIG. 1 showing a front view of a blood purification device 1 and FIGS. 2 to 6 showing a fluid delivery circuit of the blood purification device 1, explaining fluid delivery operations for priming in the blood purification device 1, an embodiment is described below.

Here, the term "priming" refers to a process including causing priming fluid to flow to a blood purifier 2 via a blood circuit 3 with the blood circuit 3 and a dialysate circuit 4 connected to the blood purifier 2 before performing blood purification treatment such as hemodialysis to remove air in the hollow fiber membrane 2B included in the blood purifier 2 and the blood circuit 3 and causing dialysate to flow to the blood purifier 2 via the dialysate circuit 4 to remove air around the hollow fiber membrane 2B within a body casing 2A.

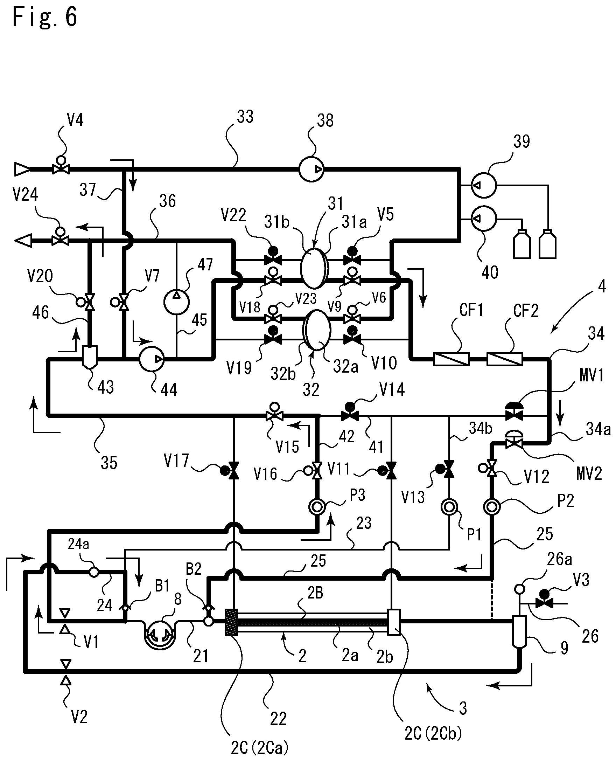

In the blood purification device 1 in this embodiment, dialysate can be supplied from the dialysate circuit 4 to the blood circuit 3 to infuse the dialysate during blood purification treatment, and post-infusion (post-dilution method, see FIG. 2), which supplies dialysate to a downstream side of the blood purifier 2, or pre-infusion (pre-dilution method, see FIG. 6), which supplies dialysate to an upstream side of the blood purifier 2, can be selected.

The blood purification device 1 includes the blood purifier 2 that performs blood purification, the blood circuit 3 having an arterial passage 21 and a venous passage 22 connected to the blood purifier 2, and the dialysate circuit 4 having a dialysate supply passage 34 and a dialysate discharge passage 35 connected to the blood purifier 2, and operations of various open/close valves and pumps provided in the blood purification device 1 are controlled by control device 5, which includes a microcomputer, personal computer, and so on.

Referring to FIG. 1, the blood circuit 3 is mounted on a front panel 6a of a body unit 6, which constitutes the blood purification device 1, whereas the dialysate circuit 4 is mostly contained inside the body unit 6.

The control device 5 is located above the body unit 6, and the control device 5 is equipped with a touch panel 5a for displaying various settings and the progress of blood purification treatment as well as for performing necessary operations, and a purifier holder 7 is provided on a side portion of the body unit 6 to hold the blood purifier 2 such that blood and dialysate can circulate up and down.

The front panel 6a of the body unit 6 has a blood pump 8 for delivering blood in the blood circuit 3, and the blood pump 8 is installed in a middle portion of the arterial passage 21 by attaching a piping tube that constitutes the arterial passage 21 in the blood circuit 3.

An arterial side clamp V1 is provided below the blood pump 8 on the front panel 6a, which can hold the piping tube of the arterial passage 21.

In parallel with the blood pump 8, a chamber holding unit 27 is further provided to hold an air trap chamber 9 in the venous passage 22 of the blood circuit 3, and a liquid level sensor 28 is provided below the chamber holding unit 27 to detect the height of the liquid level of the held air trap chamber 9.

Below the chamber holding unit 27 and the liquid level sensor 28, a venous side clamp V2 is further provided, which can hold the piping tube of the venous passage 22.

Furthermore, below the blood pump 8 on the front panel 6a of the body unit 6, a supply port P1 is provided, which is communicated to the dialysate supply passage 34 of the dialysate circuit 4 to connect the blood circuit 3 to the dialysate circuit 4 inside the body unit 6, below the chamber holding unit 27, an infusion port P2 is provided, which is communicated to the dialysate supply passage 34, and, below the supply port P1, a discharge port P3 is provided, which is communicated to the dialysate discharge passage 35 of the dialysate circuit 4. Also, above the chamber holding unit 27 on the front panel 6a, an atmospheric release port P4 is provided to be connected to an air trap chamber 9.

For each of the ports P1 to P3, the configuration described in Patent No. 5920575 can be used, for example, with a lid member L rotatably provided on the front panel 6a corresponding to each of the ports P1 to P3.

This configuration allows the lid member L to be rotated to expose each of the ports P1 to P3 to the outside and connect the corresponding passage of the blood circuit 3 when the blood circuit 3 is installed, and allows the connector 21a of the arterial passage 21 to be removed from the discharge port P3 during blood purification treatment and to be covered with the lid member L not to be exposed to the outside.

The blood purifier 2 is entirely made of resin, and includes a hollow fiber membrane 2B having many bundled hollow fibers inside a transparent cylindrical body casing 2A, and the interior of each of the hollow fibers of the hollow fiber membrane 2B forms a blood passage 2a through which blood circulates, and the surroundings of the hollow fiber membrane 2B inside the body casing 2A form a dialysate passage 2b through which dialysate circulates.

Both ends of the body casing 2A are closed with a header 2C, and an arterial side header 2Ca, which has a blood inlet to which the arterial passage 21 is connected, is colored red, and a venous side header 2Cb, which has a blood outlet to which the venous passage 22 is connected, is colored blue, communicating the ends of the hollow fiber membrane 2B to the arterial passage 21 and venous passage 22, respectively.

A dialysate outflow port 2D is provided at a position on an outer peripheral surface of the body casing 2A adjacent to the header 2C such that the dialysate supply passage 34 is connected to the dialysate inlet 2Da formed adjacent to the venous side header 2Cb, and the dialysate discharge passage 35 is connected to the dialysate outlet 2Db formed adjacent to the arterial side header 2Ca, communicating to the dialysate passage 2b around the hollow fiber membrane 2B inside the body casing 2A.

In the blood purification device 1 according to the present invention, the blood purifier 2 is held in the purifier holder 7 of the body unit 6 such that the arterial side header 2Ca is located below, and accordingly, during blood purification treatment, the blood de-bleeding from a patient in the arterial passage 21 is caused to flow through the blood passage 2a in the hollow fiber membrane 2B from below to above (from left to right in FIGS. 2 to 6) and is discharged to the venous passage 22.

The dialysate supplied from the dialysate supply passage 34 is caused to flow through the dialysate passage 2b around the hollow fiber membrane 2B from above to below (from right to left in FIGS. 2 to 6) in the opposite direction of the blood flow and is discharged to the dialysate discharge passage 35.

Such arterial passage 21, hollow fiber membrane 2B of the blood purifier 2, and venous passage 22 form a blood circulation passage.

Also, during priming, like during blood purification treatment, the blood purifier 2 is held in the purifier holder 7 with the arterial side header 2Ca facing downward, so that the dialysate as the priming fluid circulates through the blood passage 2a in the hollow fiber membrane 2B from below to above.

The distal end of the arterial passage 21 has an open end with a connector 21a to which a puncture needle is attached for treatment, and from the connector 21a to the part where it is attached to the blood pump 8, a supply passage 23 that supplies dialysate from the dialysate circuit 4 during the priming and blood return operations after blood purification treatment is branched.

The blood pump 8 is configured by a so-called tube pump, in which a plurality of rollers r are arranged at equal intervals around an outer circumference of a rotating rotor R, and, as the rotor R rotates, the rollers r successively squeeze the piping tubes that constitute the arterial passage 21 around the rotor R so that liquid is delivered in such a way that it is drawn into the piping tube and pushed out.

In this embodiment, the direction of delivering liquid from the connector 21a to the blood purifier 2 in the arterial passage 21 is the forward direction, and the rotation of the rotor R of the blood pump 8 in the forward direction is the forward rotation, and conversely, the direction of delivering liquid from the blood purifier 2 to the connector 21a is the reverse direction, and the rotation of the rotor R of the blood pump 8 in the reverse direction is the reverse rotation, and the forward direction is the downstream side and the reverse direction is the upstream side of the blood pump 8, assuming that the blood pump 8 delivering liquid in the forward direction is a standard operation.

During priming, the connector 21a of the arterial passage 21 is connected to the discharge port P3 provided on the front panel 6a of the body unit 6 such that the dialysate flowing through the blood circuit 3 is discharged into the dialysate discharge passage 35.

The arterial side clamp V1 is designed to hold the piping tube of the arterial passage 21 between a connection point 21P of the supply passage 23 and the connector 21a, and the arterial passage 21 is automatically opened and closed by drive device, not shown, controlled by the control device 5.

The supply passage 23 is used during the priming and blood return operations to supply dialysate flowing in the dialysate supply passage 34 to the blood circuit 3.

The supply passage 23 is provided integrally with the blood circuit 3 and is connected to the dialysate supply passage 34 via the supply port P1.

The distal end of the supply passage 23 has a connector 23a to connect to the supply port P1, which is branched with a check valve B1 at the base to block the inflow from the arterial passage 21.

In addition, a branch piping 24 as a venous passage connection part according to the present invention is provided branched in the middle of the supply passage 23, and a connection part 24a is provided at the distal end of the branch piping 24 to which a connector 22a provided at the distal end of the venous passage 22 can be connected, and when the branch piping 24 is not connected to the connector 22a, the branch piping 24 is closed with an occlusion clamp 24b.

The connector 22a at the distal end of the venous passage 22 is an open end to which a puncture needle is attached for blood purification treatment. The venous side clamp V2 holds the piping tube of the venous passage 22 between the air trap chamber 9 and the connector 22a, and the venous passage 22 is automatically opened and closed by drive device, not shown, controlled by the control device 5.

Between the blood purifier 2 and the air trap chamber 9 in the venous passage 22, an infusion passage 25 for post-infusion is branched and connected to the dialysate supply passage 34 via the infusion port P2.

The distal end of the infusion passage 25 is connected to the infusion port P2 at the open end with connector 25a, and is branched with a check valve B2 at the base to block the inflow from the venous passage 22.

As shown in FIG. 1, the air trap chamber 9 has a cylindrical transparent container 9a, a cap 9b sealing an upper part of the container 9a, and a filter 9c provided at a bottom part of the container 9a, and the cap 9b has an air piping tube 9d for connection to the atmospheric release port P4.

The air trap chamber 9 is provided in a middle part of the venous passage 22, with a downstream piping tube of the venous passage 22 connected to a lower part of the container 9a, and an upstream piping tube of the venous passage 22 connected to an upper part of the side and in communication with them.

This configuration allows liquid to flow in from the upper part of container 9a and out from the lower part, trapping air that flows in with the liquid by releasing it into the container 9a, and the filter 9c traps clots that flow in with the blood.

The atmospheric release port P4 is communicated to a gas passage 26 and open to the outside, and the gas passage 26 can be opened and closed by an atmospheric release valve V3 provided therein. Between the atmospheric release port P4 and the atmospheric release valve V3, a pressure gauge 26a is provided to measure the venous pressure in the blood circuit 3.

Below the chamber holding unit 27, a liquid level sensor 28 is provided that detects when the liquid level has dropped to the level of the filter 9c for the air trap chamber 9 held in the chamber holding unit 27.

The liquid level sensor 28 monitors the liquid level height in the air trap chamber 9, and when it detects that the liquid level has dropped due to air accumulation in the air trap chamber 9 during priming, the control device 5 opens the atmospheric release valve V3 to release the air trap chamber 9 to the atmosphere, thereby raising the liquid level. The atmospheric release valve V3 is closed when the liquid level rises to a predetermined height.

Referring to FIG. 2, the dialysate circuit 4 includes first and second dialysate chambers 31 and 32 with supply compartments 31a and 32a and recovery compartments 31b and 32b formed inside by diaphragms, a feed passage 33 that supplies cleaned dialysate water to the supply compartments 31a and 32a, a dialysate supply passage 34 that supplies fresh dialysate from the supply compartments 31a and 32a to the blood purifier 2, a dialysate discharge passage 35 that discharges used dialysate from the blood purifier 2 to the recovery compartments 31b and 32b, and a drain passage 36 for draining used dialysate from the recovery compartments 31b and 32b.

In the feed passage 33, a water supply source, not shown, that supplies cleaned dialysis water is connected to an upstream side feed port, an water supply valve V4 is provided at the feed port, and a feed pump 38 is provided downstream of it.

A downstream portion of the feed passage 33 is branched in two directions and connected to the supply compartments 31a and 32a of the first and second dialysate chambers 31 and 32, and feed valves V5 and V6 are provided in the branched passages, respectively.

Also, a first bypass passage 37 branched from between the water supply valve V4 and the feed pump 38 and connected to the drain passage 36 is provided, and a seventh open/close valve V7 is provided in the first bypass passage 37.

Between the feed pump 38 and a downstream portion of the feed passage 33 branched from the feed pump 38, a B concentrate pump 39 that supplies a B concentrate and an A concentrate pump 40 that supplies an A concentrate are connected as concentrate supply device for supplying dialysis concentrate.

The A and B concentrates supplied in predetermined amounts by the concentrate pumps 39 and 40 are caused to flow through the feed passage 33 together with the dialysis water delivered by the feed pump 38, and flow into the supply compartments 31a and 32a of the first and second dialysate chambers 31 and 32.

These A and B concentrates and dialysis water are mixed inside the supply compartments 31a and 32a to prepare a dialysis of a predetermined concentration.

The upstream portion of the dialysate supply passage 34 branches in two directions, and the branched upstream portions are connected to the supply compartments 31a and 32a of the first and second dialysate chambers 31 and 32, respectively, and the downstream end is connected to a dialysate inlet 2Da adjacent to the venous side header 2Cb of the blood purifier 2 and is communicated to the dialysate passage 2b within the blood purifier 2.

Furthermore, supply valves V9 and V10 are provided in the branched upstream portions of the dialysate supply passage 34, respectively, and two filters CF1 and CF2, which capture endotoxin in the dialysate and clean the dialysate, are provided adjacent to each other downstream of the upstream portions, and a supply port P1 and an infusion port P2 are located downstream of these filters CF1 and CF2 so that the cleaned dialysate is supplied to the blood circuit 3.

An infusion branch passage 34a is branched at a position downstream of the filters CF1 and CF2, and first flow restriction device MV1 is provided in the dialysate supply passage 34 downstream of the branch position, and second flow restriction device MV2 and a 12th open/close valve V12 are provided in the infusion branch passage 34a, and an infusion port P2 is provided at the distal end of the infusion branch passage 34a to allow the dialysate to flow out of the dialysate supply passage 34.

When performing the infusion during blood purification treatment, the control device 5 adjusts the opening degrees of the first flow restriction device MV1 and second flow restriction device MV2 and opens the 12th open/close valve V12, so that a part of the dialysate flowing in the dialysate supply passage 34 is caused to flow to the infusion branch passage 34a and is supplied from the infusion passage 25 to either the venous passage 22 or the arterial passage 21 through the infusion port P2.

Such flow control for supplying supplemental fluid from the dialysate circuit to the blood circuit is described, for example, in Japanese Laid-Open Patent Application No. 2021-062067 and is publicly known in the past.

A supply branch passage 34b is branched downstream of the first flow restriction device MV1 in the dialysate supply passage 34, and a 13th open/close valve V13 is provided in the supply branch passage 34b, and a supply port P1 is provided at the distal end of the supply branch passage 34b, so that the dialysate can be caused to flow out of the dialysate supply passage 34. Furthermore, an 11th open/close valve V11 is provided in the dialysate supply passage 34 downstream of the branch position of the supply branch passage 34b, and the downstream end is connected to the blood purifier 2.

From between the branch position of the supply branch passage 34b and the 11th open/close valve V11, a second bypass passage 41 branches off and is connected to the dialysate discharge passage 35, and the second bypass passage 41 is equipped with a 14th open/close valve V14 and a 15th open/close valve V15.

A discharge passage 42 is provided branching off from between the 14th open/close valve V14 and 15th open/close valve V15, and a 16th open/close valve V16 is provided in the discharge passage 42, and a discharge port P3 is provided at the distal end of the discharge passage 42 to allow dialysate to flow into the dialysate discharge passage 35 via the second bypass passage 41.

The dialysate discharge passage 35 has its upstream end connected to the blood purifier 2. In the dialysate discharge passage 35, a 17th open/close valve V17 is provided upstream from the connection position of the second bypass passage 41, and a degassing tank 43 to remove air bubbles in the dialysate and a dialysate pump 44 to deliver dialysate are provided downstream in that order.

A downstream portion of the dialysate discharge passage 35 branches off in two directions, and the branched downstream portions are connected to the recovery compartments 31b and 32b of the first and second dialysate chambers 31 and 32, and recovery valves V18 and V19 are provided at these branched portions.

Furthermore, the drain passage 36 with its upstream portion branching in two directions is connected to the recovery compartments 31b and 32b of the first and second dialysate chambers 31 and 32, and drain valves V22 and V23 are provided at the branched upstream portions, and an open/close valve V24 is provided at the downstream outlet.

The degassing tank 43 is provided with an exhaust passage 46 whose distal end is connected to the drain passage 36, and the exhaust passage 46 is provided with a 20th open/close valve V20.

The degassing tank 43 is conventionally publicly known, and when used dialysate flows into the degassing tank 43 during blood purification treatment, the air that flows in with the used dialysate is separated and discharged through the exhaust passage 46 from the drain passage 36, and excess dialysate can be caused to overflow from the dialysate discharge passage 35 into the drain passage 36.

The first bypass passage 37 is connected between the degassing tank 43 and the dialysate pump 44 and is used to refill the dialysate discharge passage 35 with dialysate water from the feed passage 33 during priming.

In other words, by opening the seventh open/close valve V7, the dialysate pump 44 can draw dialysate water from the feed passage 33 into the dialysate discharge passage 35 to be contained in the recovery compartments 31b and 32b.

A de-watering passage 45 is connected downstream the dialysate pump 44 in the dialysate discharge passage 35 to discharge used dialysate from the dialysate discharge passage 35 to the drain passage 36 without going through the recovery compartments 31b and 32b, and a de-watering pump 47 is provided in the de-watering passage 45.

During blood purification treatment, the de-watering pump 47 is actuated to create a differential pressure between a dialysate passage 2b and a blood passage 2a in the blood purifier 2, which moves the water in the blood through the hollow fiber membrane 2B to the dialysate circuit 4 side for removal.

The priming method of the blood circulation passage in the blood purification device 1 having the aforementioned configuration is explained below with reference to FIGS. 2 to 5. Here, for performing post-infusion in blood purification treatment, the blood circuit 3 is used in which the infusion passage 25 is branched from the venous passage 22.

In each of the figures, the parts through which dialysate and dialysis water are caused to flow are indicated with thick lines. However, the flow of dialysate through the dialysate circuit 4 shall be explained with thick lines for the flow of liquid resulting from priming, while thick lines shall be omitted for the flow of dialysate during normal operation, such as during blood purification treatment.

Furthermore, unless otherwise indicated, the black valves and clamps in the figures are shown closed and the white ones are shown open.

Before priming, the blood purifier 2 and blood circuit 3 are attached to the blood purification device 1.

As shown in FIG. 1, the medical personnel holds the piping tube that constitutes the blood circuit 3 in the holder, not shown, provided on the front panel 6a of the body unit 6, connects the connector 21a provided at the distal end portion of the arterial passage 21 to the discharge port P3, and attaches the piping tube constituting the arterial passage 21 to the blood pump 8 and the arterial side clamp V1. The connector 23a provided at the distal end portion of the supply passage 23, which is branched from the arterial passage 21, is connected to the supply port P1.

Furthermore, the medical personnel connects the connector 22a provided at the distal end portion of the venous passage 22 to the connection part 24a of the branch piping 24 branched from the supply passage 23 and opens the occlusion clamp 24b of the branch piping 24. The piping tube that constitutes the venous passage 22 is attached to the venous side clamp V2.

The medical personnel also attaches the air trap chamber 9 to the chamber holding unit 27 and connects the air piping tube 9d to the atmospheric release port P4. In addition, the connector 25a provided at the distal end portion of the infusion passage 25 is connected to the infusion port P2.

The medical personnel then holds the blood purifier 2 in the purifier holder 7 with the red arterial side header 2Ca facing downward and the blue venous side header 2Cb facing upward and connects the arterial passage 21 to the blood inlet of the arterial side header 2Ca and the venous passage 22 to the blood outlet of the venous side header 2Cb.

The dialysate discharge passage 35 of the dialysate circuit 4 is connected to the dialysate outlet 2Db adjacent to the arterial side header 2Ca, and the dialysate supply passage 34 is connected to the dialysate inlet 2Da adjacent to the venous side header 2Cb.

Once the blood purifier 2 and the blood circuit 3 are attached to the body unit 6 in this manner, the medical personnel operates the touch panel 5a to instruct to start priming.

In the following description, a detailed explanation of the dialysate and dialysate water delivering operation in the dialysate circuit 4 is omitted, but the condition in FIG. 2 is that in the first dialysate chamber 31, the supply valve V9 in the supply compartment 31a and the recovery valve V18 in the recovery compartment 31b are open and the dialysate prepared in the supply compartment 31a is delivered into the dialysate supply passage 34, and, in the second dialysate chamber 32, the supply valve V6 in the supply compartment 32a and the drain valve V23 in the recovery compartment 32b are open and the dialysate is being prepared in the supply compartment 32a.

In the feed passage 33, the water supply valve V4 is open and the feed pump 38 is actuated and the seventh open/close valve V7 of the first bypass passage 37 provided between the feed passage 33 and the dialysate discharge passage 35 is open.

In other words, in this condition, when the dialysate pump 44 in the dialysate discharge passage 35 delivers the dialysate, the dialysate water from the feed passage 33 circulates in the first bypass passage 37 and flows into the dialysate discharge passage 35 and into the recovery compartment 31b of the first dialysate chamber 31.

Then, the volume of the recovery compartment 31b expands due to the inflow of dialysate water, and the volume of the supply compartment 31a shrinks as dialysate is delivered from the supply compartment 31a to the dialysate supply passage 34.

Meanwhile, in the second dialysate chamber 32, dialysate is prepared by mixing predetermined amounts of dialysate water, dialysate B concentrate, and dialysate A concentrate in the supply compartment 32a by actuating the feed pump 38 and the B concentrate pump 39 and A concentrate pump 40, and the contained dialysate water is discharged from the recovery compartment 32b into the drain passage 36.

During blood purification treatment, since the path from the supply compartments 31a and 32a to the recovery compartments 31b and 32b via the blood purifier 2 is filled with dialysate, the dialysate flows from the supply compartments 31a and 32a via the dialysate supply passage 34, the dialysate passage 2b of blood purifier 2 and the dialysate discharge passage 35 to the recovery compartments 31b and 32b when the dialysate pump 44 is actuated.

On the other hand, at the start of priming, the blood circulation passage consisting of the blood purifier 2 and the blood circuit 3 is not filled with dialysate, and so even if the dialysate pump 44 is actuated as with during treatment, dialysate is not contained in the recovery compartments 31b and 32b and cannot be delivered from the supply compartments 31a and 32a.

Therefore, in this embodiment, the seventh open/close valve V7 of the first bypass passage 37 is opened to supply dialysis water from the first bypass passage 37 to the recovery compartments 31b and 32b via the dialysate discharge passage 35, so that the dialysis can be delivered from the supply compartments 31a and 32a.

This allows the dialysate to flow through the blood purifier 2 and blood circuit 3, but when the dialysate is caused to flow throughout the flow paths of the blood purifier 2 and blood circuit 3, a surplus then occurs. The 20th open/close valve V20 of the exhaust passage 46 is opened to allow the excess dialysate to flow from the exhaust passage 46 of the degassing tank 43 into the drain passage 36.

The dialysate supply operation by the dialysate supply device is such that the dialysate can be supplied continuously by alternately preparing and draining and supplying and recovering dialysate in the first and second dialysate chambers 31 and 32.

These feed pump 38, concentrate supply device, first dialysate chamber 31, second dialysate chamber 32, and dialysate pump 44 constitute the dialysate supply device of the present invention, and their operations are controlled by the control device 5.

Once priming is started, priming the infusion passage 25 shown in FIG. 2 is first performed.

The control device 5 adjusts the flow rate such that dialysate flows into the infusion branch passage 34a by means of the first flow restriction device MV1 in the dialysate supply passage 34 and the second flow restriction device MV2 in the infusion branch passage 34a and closes the 11th open/close valve V11, the 13th open/close valve V13 in the supply branch passage 34b and the 14th open/close valve V14 of the second bypass passage 41.

The control device 5 also opens the arterial side clamp V1 of the arterial passage 21 and the venous side clamp V2 of the venous passage 22 and also closes the atmospheric release valve V3 of the gas passage 26 to stop the blood pump 8.

As a result, the dialysate flowing through the dialysate supply passage 34 flows from the infusion branch passage 34a through the infusion passage 25 and into the venous passage 22, and priming the infusion passage 25 is performed.

The dialysate that flows into between the blood purifier 2 and the air trap chamber 9 in the venous passage 22 does not flow toward the blood purifier 2 but flows toward the air trap chamber 9 because the blood pump 8 is stopped and the rollers r compress the piping tube in the arterial passage 21 and blocks the passage.

The dialysate that flows into the air trap chamber 9 then further flows through the venous passage 22 and then flows into the supply passage 23 via the branch piping 24.

Since the 13th open/close valve V13 of the supply passage 23 is closed, the dialysate flows into the arterial passage 21 through the check valve B1 in the supply passage 23, and since the blood pump 8 is stopped and the passage is closed, the dialysate is caused to flow through the arterial passage 21 toward the discharge port P3.

The control device also closes the 17th open/close valve V17 in the dialysate discharge passage 35, opens the 16th open/close valve V16 in the discharge passage 42 and the 15th open/close valve V15 in the second bypass passage 41 and opens the 20th open/close valve V20 in the exhaust passage 46 connected to the degassing tank 43.

As a result, the dialysate flowing through the arterial passage 21 flows into the discharge passage 42 via the discharge port P3 and flows out into the dialysate discharge passage 35 via the second bypass passage 41 and is either contained in the recovery compartment 31b by the action of the dialysate pump 44 or discharged from the degassing tank 43 to the drain passage 36 via the exhaust passage 46.

The control device 5 continues these operations for a predetermined time, which fills the infusion passage 25 with dialysate and completes the priming of the infusion passage 25.

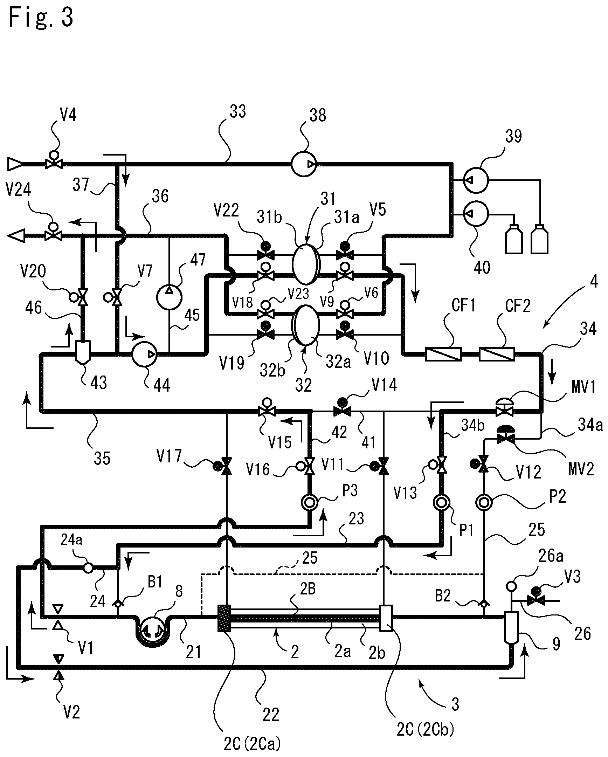

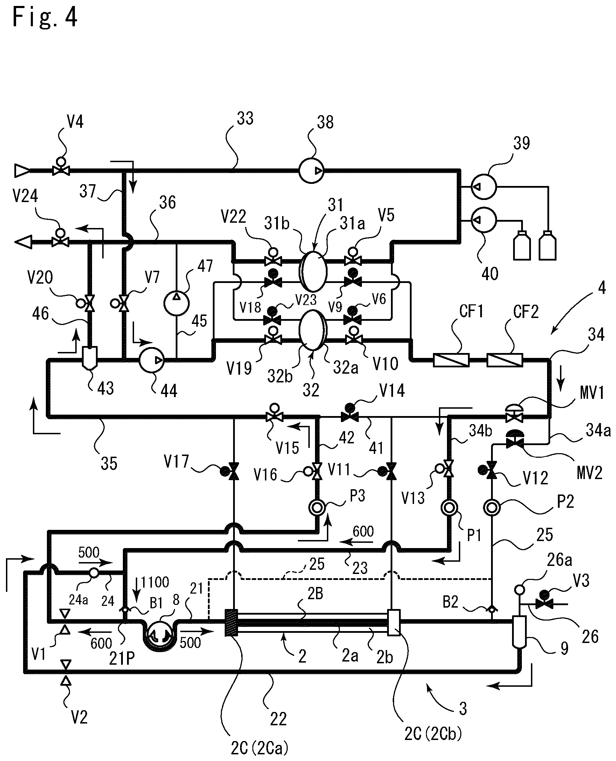

FIG. 3 shows an operation of removing air from the filter 9c provided in the air trap chamber 9 of the venous passage 22, which is done before the simultaneous priming of the arterial passage 21 and the venous passage 22 of the blood circuit 3 shown in FIG. 4.

During priming of the infusion passage 25 shown in FIG. 2, dialysate flows into the air trap chamber 9 and passes through the filter 9c provided at the bottom, but air bubbles attached to said filter 9c cannot be easily removed.

The control device 5 adjusts the first flow restriction device MV1 in the dialysate supply passage 34 and the second flow restriction device MV2 in the infusion branch passage 34a such that there is no flow into the infusion branch passage 34a, closes the 12th open/close valve V12 in the infusion branch passage 34a and opens the 13th open/close valve V13 in the supply branch passage 34b.

Furthermore, the control device 5 reverses the blood pump 8 in the arterial passage 21 and leaves the arterial side clamp V1 open, while the venous side clamp V2 in the venous passage 22 is opened and closed at predetermined intervals.

Then, the dialysate flowing through the dialysate supply passage 34 flows through the supply passage 23 from the supply branch passage 34b and flows into the venous passage 22 through the branch piping 24 instead of into the arterial passage 21 due to the pressure loss of the check valve B1.

Thus, the check valve B1 functions as flow prevention device for preventing flow of dialysate from the supply passage 23 to the arterial passage 21, but the flow prevention device is not limited to the check valve, and can be open/close valves or orifices, as long as the means can be used to prevent the flow of dialysate when the blood pump 8 is reversed.

When the dialysate flowing through the venous passage 22 in the opposite direction to that in performing treatment flows in from the lower side of the air trap chamber 9, air bubbles attached to the filter 9c are removed by the backwash action caused by the reverse directional inflow when passing through the filter 9c.

In conjunction with this, opening and closing the venous side clamp V2 cause pressure fluctuations in the flowing dialysate, and the pulsation enables efficient removal of air bubbles from the filter 9c.

The dialysate discharged from the upper side of the air trap chamber 9 then passes through the blood purifier 2, is fed to the reversing blood pump 8 for further reverse flow through the arterial passage 21, and then flows out through the discharge port P3 from the discharge passage 42 to the dialysate discharge passage 35.

Here, since the check valve B1 is provided in the supply passage 23B, the dialysate flowing through the arterial passage 21 does not flow into the supply passage 23.

FIG. 4 shows simultaneous priming of the arterial passage 21 and the venous passage 22 of the blood circuit 3.

In this operation in FIG. 4, the dialysate is supplied from the dialysate supply passage 34 to the arterial passage 21 via the supply passage 23, and the supplied dialysate is caused to flow by being divided at the branching point 21P of the arterial passage 21 with the supply passage 23 in the direction of the blood purifier 2 (forward direction) and the direction of the distal end portion connected to the discharge port P3 (reverse direction), and the dialysate is caused to flow to the blood purifier 2 is caused to flow through the venous passage 22 to merge with the supply passage 23 again from the distal end of the venous passage 22 through the branch passage 24.

Specifically, the control device 5 switches the blood pump 8, which had been reversed, from the state shown in FIG. 3 to the forward rotation to deliver fluid in the forward direction at a predetermined flow rate.

The dialysate flowing into the branching point 21P of the arterial passage 21 with the supply passage 23 flows in the forward direction, that is, toward the blood purifier 2, by the forward rotating blood pump 8 to form a circulation passage consisting of a part of the arterial passage 21, the hollow fiber membrane 2B of the blood purifier 2, the venous passage 22, the branch passage 24 and a part of the supply passage 23. Dialysate flowing from the branch passage 24 into the supply passage 23 merges with dialysate supplied from dialysate supply passage 34 and flowing through the supply passage 23 and flows into the arterial passage 21.

The flow rate of dialysate flowing from the branch passage 24 into the supply passage 23 is due to the set flow rate of the blood pump 8, and the flow rate of dialysate supplied from the dialysate supply passage 34 is due to the set flow rate of the dialysate pump 44, which constitutes the dialysate supply device, and so the flow rate of dialysate flowing from the supply passage 23 into the arterial passage 21 is the total flow rate.

Therefore, at the branching point 21P, dialysate for the set flow rate of the blood pump 8 flows in the forward direction, and the dialysate for the remaining flow rate flows in the reverse direction and is discharged into the dialysate discharge passage 35.

For example, at the position where the branch piping 24 merges with the supply passage 23, the 600 ml/min dialysate supplied from the dialysate supply passage 34 and the 500 ml/min dialysate circulated by the blood pump 8 in the circulation passage will merge, resulting in a total of 1100 ml/min dialysate, which is supplied to the arterial passage 21.

Since the blood pump 8 then delivers dialysate in the forward direction at a flow rate of 500 ml/min, at the branch point P21 of the supply passage 23 in the arterial passage 21, the 500 ml/min dialysate flows in the forward direction to merge with the supply passage 23 again along the circulation passage, and the remaining 600 ml/min dialysate is discharged into the dialysate discharge passage 35 through the discharge passage 42 after flowing in the reverse direction in the arterial passage 21.

In this way, the dialysate can be caused to flow throughout the entire passage of the arterial passage 21 and venous passage 22, which can be primed simultaneously. The set flow rates of the blood pump 8 and dialysate pump 44 (dialysate supply device) are not limited to the dialysate pump 44 (dialysate supply device) exceeding the blood pump 8 as in the example, but may be the same flow rate or the blood pump 8 exceeding the dialysate pump 44.

At this time, if the amount of air released in the air trap chamber 9 increases and the liquid level drops to the level detected by the liquid level sensor 28, the control device 5 releases the air in the air trap chamber 9 by opening the atmospheric release valve V3 for a predetermined period of time. When the air is released, the atmospheric release valve V3 is closed again, and the liquid level in the air trap chamber 9 returns to a predetermined height.

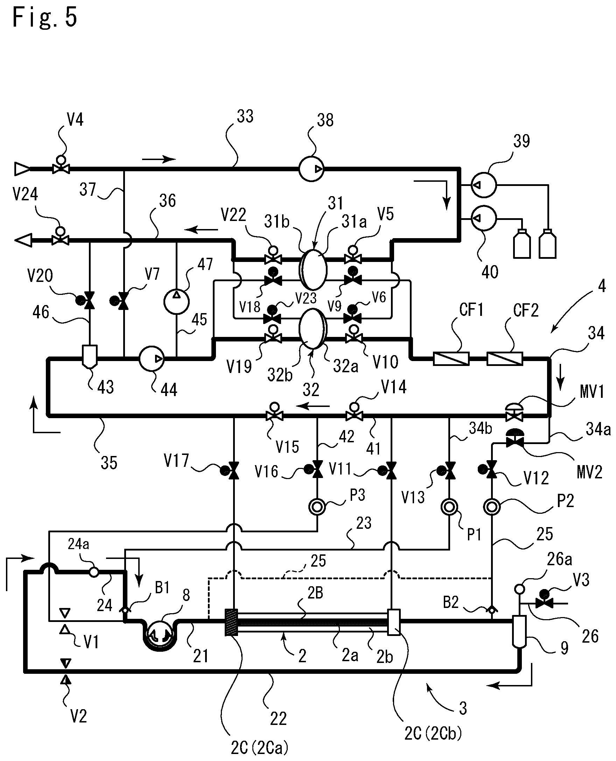

FIG. 5 shows priming of the hollow fiber membrane 2B of the blood purifier 2, which is done after the simultaneous priming of the arterial passage 21 and the venous passage 22 of the blood circuit 3 shown in FIG. 4.

For the dialysate circuit 4, from the state of priming of the blood circuit 3 shown in FIG. 4, the control device 5 closes the 13th open/close valve V13 in the supply branch passage 34b and the 16th open/close valve V16 in the discharge passage 42, and opens the 14th open/close valve V14 in the second bypass passage. Also, the seventh open/close valve V7 in the first bypass passage 37 and the 20th open/close valve V20 in the exhaust passage 46 of the degassing tank 43 are closed.

This means that the dialysate supply device only recovers and drains the dialysate prepared in the supply compartments 31a and 32a into the recovery compartments 31b and 32b, and the supply of dialysate from the dialysate supply passage 34 to the blood circuit 3 is stopped.

For the blood circuit 3, the control device 5 causes the blood pump 8 to rotate forward to deliver fluid in the forward direction and opens and closes the venous side clamp V2 of the venous passage 22 at predetermined intervals.

As a result, the dialysate delivered by the blood pump 8 flows from the arterial passage 21 into the downward-facing arterial side header 2Ca of the blood purifier 2, flows through the hollow fiber membrane 2B, flows from the upward-facing venous side header 2Cb to the venous passage 22, and then circulates through the branch piping 24 and supply passage 23 to the arterial passage 21.

In the meantime, by opening and closing the venous side clamp V2, the dialysate is pulsed, and air attached to the hollow fiber membrane 2B is caused to flow out into the venous passage 22 and is recovered by the air trap chamber 9.

The control device 5 continues the circulation of dialysate for a predetermined period of time while releasing air to the outside by opening the atmospheric release valve V3 while detecting with the liquid level sensor 28 that the air volume in the air trap chamber 9 has increased.

Since priming is performed by circulating dialysate in this manner, the use of dialysate can be suppressed compared to priming while discharging the supplied dialysate.

As described above, the blood purification device 1 and the method for priming blood circulation passages in the embodiment above make it possible to prime the hollow fiber membrane 2B of the blood purifier 2 and the arterial passage 21 and venous passage 22 of the blood circuit 3 simultaneously.

Specifically, the distal end portion of the arterial passage 21 is connected to the discharge port P3 such that it communicates with the dialysate discharge passage 35, and the distal end portion of the venous passage 22 is connected to the supply passage 23 to form a circulation passage and actuate the blood pump 8 to deliver fluid in the forward direction.

As a result, the dialysate supplied from the dialysate supply passage 34 partially circulates in the circulation passage, while the remaining dialysate circulates in the reverse direction through the arterial passage 21 and is discharged from the discharge port P3, thus allowing to simultaneously prime the whole of the hollow fiber membrane 2B of the blood purifier 2, arterial passage 21 and venous passage 22.

Following this, priming can be performed by circulating the dialysate through the circulation passage through the formed blood purifier with the supply of dialysate from the dialysate supply passage 34 stopped, thereby sufficiently priming the hollow fiber membrane 2B in the blood purifier without using new dialysate so that the use of dialysate can be suppressed.

In the present invention, the blood purifier 2 is used for blood purification treatment by delivering blood from below to above with the arterial side header 2Ca facing downward and the venous side header 2Cb facing upward, and priming of the hollow fiber membrane 2B of the blood purifier 2 is performed by supplying dialysate to the arterial passage 21 and causing the dialysate to flow from below to above. Therefore, there is no need to invert the blood purifier 2 when performing blood purification treatment after priming, which can reduce the workload on medical personnel.

Furthermore, according to this embodiment, even when selecting post-infusion using a blood circuit in which the infusion passage 25 is branched from the venous passage 22, or when selecting pre-infusion using a blood circuit in which a supply passage 23 is branched from the arterial passage 21, priming of the blood purifier 2 and the blood circuit 3 can be performed by the same operation by supplying dialysate from the supply passage 23 branched from the arterial passage 21. However, the priming of the infusion passage 25 is different, and so a method for priming the infusion passage 25 in a case where pre-infusion is selected is explained below.

FIG. 6 shows priming operations when using the blood circuit 3 with the infusion passage 25 branched from the arterial passage 21 for performing pre-infusion, corresponding to the priming operations on the infusion passage 25 in the blood circuit 3 in a case where post-infusion is performed as shown in FIG. 2.

When the infusion passage 25 is branched from the arterial passage 21, the dialysate supplied from the dialysate supply passage 34 flows between the blood pump 8 and the blood purifier 2 in the arterial passage 21 after flowing in the infusion passage 25.

Even in this case, since the arterial passage 21 is blocked by the blood pump 8 being stopped, the dialysate that flows into the arterial passage 21 passes through the hollow fiber membrane 2B of the blood purifier 2, flows through the venous passage 22, flows into the supply passage 23 through the branch piping 24, flows from the arterial passage 21 through the discharge port P3 into the discharge passage 42 and is discharged into the dialysate discharge passage 35.

The subsequent priming operations can be performed in the same manner as shown in FIGS. 3 to 5, as with the case of post-infusion using the blood circuit 3, in which the infusion passage 25 is branched from the venous passage 22, because the infusion passage 25 is not used to supply dialysis.

Thus, according to this embodiment, priming of the blood circulation passage consisting of the blood purifier 2 and the blood circuit 3 can be performed by the same operations, regardless of whether post-infusion or pre-infusion is performed.

It should be noted that the priming method of this embodiment can be used to prime, with the same operations, the blood circuit 3 that is not equipped with the infusion passage 25.

While the aforementioned embodiment describes a so-called personal blood purification device in which the blood purification device 1 is equipped with dialysate concentrate supply device and that prepares dialysate by mixing supplied dialysate water and dialysate concentrate, it can also be a so-called console-type dialysis monitoring device that receives supply of dialysate prepared by a dialysate supply device for multiple patients where the blood purification device 1 does not include dialysate concentrate supply device.

### Reference Signs List

1: blood purification device, 2: blood purifier, 3: blood circuit, 4: dialysate circuit, 8: blood pump 9 air trap chamber, 21: arterial passage, 22: venous passage, 23: supply passage, 24: branch piping (venous passage connection part), 25: infusion passage, 34: dialysate supply passage, 35: dialysate discharge passage, 42: discharge passage, P1: supply port, P2: infusion port, P3: discharge port

## Claims

1. A blood purification device having a blood circuit having an arterial passage and a venous passage connected to a blood purifier, a dialysate circuit having a dialysate supply passage and a dialysate discharge passage connected to the blood purifier, dialysate supply device for supplying dialysate to the blood purifier via the dialysate supply passage, a blood pump installed in the arterial passage to pump blood to the blood purifier, and control device for controlling operation of the dialysate supply device and the blood pump, the blood purification device being **characterized by** comprising:
a supply port for causing dialysate to flow out of the dialysate supply passage and a discharging port for causing dialysate to flow into the dialysate discharge passage; and
a supply passage branching from an upstream side of the blood pump in the arterial passage and a venous passage connection part that is connectable to the venous passage in the middle of the supply passage,
and **characterized in that**, when priming the blood circulation passage consisting of the blood purifier and blood circuit,
with the supply passage connected to the supply port, the arterial passage connected to the discharge port, and the venous passage connected to the venous passage connection part of the supply passage,
the control device actuates the dialysate supply device and the blood pump to supply dialysate from the dialysate supply passage to the arterial passage via the supply passage and further circulates the dialysate from the arterial passage through the blood purifier and venous passage, and causes the dialysate to flow out from the arterial passage to the dialysate discharge passage.

2. The blood purification device according to claim 1, **characterized by** further comprising an infusion port for causing dialysate to flow out from the dialysate supply passage separately from the supply port; and
an infusion passage branching from either the arterial passage or the venous passage separately from the supply passage.

3. A method of priming a blood circulation passage including a blood purifier and a blood circuit in a blood purification device having the blood circuit having an arterial passage and a venous passage connected to the blood purifier, a dialysate circuit having a dialysate supply passage and a dialysate discharge passage connected to the blood purifier, dialysate supply device for supplying dialysate to the blood purifier via the dialysate supply passage, a blood pump installed in the arterial passage to pump blood to the blood purifier, and control device for controlling operation of the dialysate supply device and the blood pump, the method being **characterized by** comprising:
connecting an upstream side of the blood pump in the dialysate supply passage and the arterial passage via a supply passage, connecting the venous passage to a middle of the supply passage, and connecting the arterial passage to the dialysate discharge passage, and
supplying the dialysate from the dialysate supply passage to the arterial passage via the supply passage by the dialysate supply device and causing the dialysate to be circulated by the blood pump from the arterial passage through the blood purifier and the venous passage and causing the dialysate to be discharged from the arterial passage to the dialysate discharge passage.

4. The method of priming a blood circulation passage according to claim 3, **characterized by** further comprising: after supplying dialysate from the dialysate supply passage to the arterial passage via the supply passage by the dialysate supply device, circulating the dialysate from the arterial passage through the blood purifier and venous passage by the blood pump, and discharging the dialysate from the arterial passage to the dialysate discharge passage,
preventing flow of the dialysate from the dialysate supply passage to the supply passage and flow of the dialysate from the arterial passage to the dialysate discharge passage and circulating the dialysate by causing the dialysate to flow by the blood pump from the arterial passage through the blood purifier and venous passage.

5. The method of priming a blood circulation passage according to claim 4, **characterized by** further comprising: before supplying dialysate from the dialysate supply passage to the arterial passage via the supply passage by the dialysate supply device, circulating the dialysate from the arterial passage through the blood purifier and venous passage by the blood pump, and discharging the dialysate from the arterial passage to the dialysate discharge passage,
preventing flow of dialysate from the supply passage to the arterial passage to allow the dialysate to flow from the supply passage to the venous passage, and reversing the blood pump to deliver the dialysate in the reverse direction through the arterial passage and causing the dialysate to be circulated from the venous passage to the blood purifier and to be discharged from the arterial passage to the dialysate discharge passage.
